# EUROPÄISCHE PATENTSCHRIFT

(11) **EP 1 945 751 B1**
(45) Veröffentlichungstag und Bekanntmachung des Hinweises auf die Patenterteilung: **27.07.2011**
(21) Anmeldenummer: 06806428.6
(22) Anmeldetag: 20.10.2006
(51) Int. Cl.: C12M 1/107

(54) **FERMENTER ZUR ERZEUGUNG VON BIOGAS AUS ORGANISCHEM MATERIAL**
FERMENTER FOR PRODUCING BIOGAS FROM ORGANIC MATERIAL
FERMENTEUR POUR LA PRODUCTION DE BIOGAZ A PARTIR DE MATIERE ORGANIQUE

(30) Priorität: 11.11.2005 DE 102005054323
(43) Veröffentlichungstag der Anmeldung: 23.07.2008
(73) Patentinhaber: Gantefort, Wilhelm, 46359 Heiden (DE); Beck, Jürgen, 72108 Rottenburg (DE)
(72) Erfinder: GANTEFORT, Wilhelm, 46359 Heiden (DE)
(74) Vertreter: Michalski Hüttermann & Partner Patentanwälte
(86) Internationale Anmeldenummer: PCT/EP2006/010127
(87) Internationale Veröffentlichungsnummer: WO 2007/054193

(56) Entgegenhaltungen:
- EP-A1- 0 335 825
- EP-A2- 0 521 302
- DE-A1- 19 746 636

## Beschreibung

Die vorliegende Erfindung betrifft einen Fermenter zur Erzeugung von Biogas aus organischem Material gemäß dem Oberbegriff des Anspruchs 1.

Fermenter zur Erzeugung von Biogas stossen auf zunehmendes Interesse, seit die Diskussion um erneuerbare Energien sowie deren Förderung mehr und mehr in den Mittelpunkt der Öffentlichkeit gerückt ist. Solche Fermenter sind aus dem Einsatz in-landwirtschaftlichen Betrieben sowie kommunalen Kläranlagen bekannt. Prinzip dieser Fermenter ist, dass organisches Material in einem geschlossenen Behälter gelagert wird und die enthaltenden organischen Kohlenstoffverbindungen durch mikrobielle Aktivität zu Methangas abgebaut werden, welches aufgefangen und zur Wärme- und/oder Stromerzeugung verwendet wird. Die auf diese Weise gewonnene Energie ist nahezu CO₂-neutral, da das bei der Verbrennung freigesetzte Kohlendioxid zuvor durch pflanzliche Fotosynthese der Atmosphäre entnommen wurde.

Während in der Vergangenheit fast ausschließlich organische Abfälle zur Erzeugung von Biogas herangezogen wurden, so zum Beispiel Klärschlämme, Tierexkremente aus landwirtschaftlicher Erzeugung oder Häckselabfälle, werden in jüngerer Zeit mehr und mehr auch landwirtschaftliche Primärerzeugnisse für die Erzeugung von Biogas verwendet, da diese einen weitaus höheren Anteil an organisch gebundenem Kohlenstoff aufweisen und so einen höheren Ertrag an Methangas versprechen. Aufgrund des zunehmenden Interesses an erneuerbaren Energien rücken daher diese Produktionsmethoden mehr und mehr in den Mittelpunkt.

Im Folgenden soll zunächst der unter Sauerstoffabschluss verlaufende Gärprozess zur Erzeugung von Biogas beschrieben werden.

Der gesamte Gärprozess kann in mehrere Phasen eingeteilt werden. In der ersten Phase werden die im zu vergärenden Substrat enthaltenen Kohlenhydrate, Fette und Proteine durch fakultativ und obligat anaerobe Mikroorganismen in niedermolekulare Kohlenwasserstoffverbindungen (C₁-C₅-Körper). Kohlenhydrate werden dabei sukzessive zu Propionsäure oder Buttersäure bzw. Butanol abgebaut, Fettsäuren über den Weg der ß-Oxidation schrittweise in C₂₋Einheiten zerlegt, die als Essigsäure freigesetzt werden, und Aminosäuren nach der Stickland - Reaktion gekoppelt zu Essigsäure, Ammoniak und CO₂ abgebaut.

Diese Zwischenprodukte werden wiederum zu den methanogenen Substraten Essigsäure (CH₃COOH), Wasserstoff (H₂), Kohlensäure (H₂CO₃), Ameisensäure (HCOOH) und Methanol (CH₃OH) abgebaut.

Diese methanogenen Substrate werden wiederum von obligat anaeroben, methanbildenen (methanogenen) Bakterien der Gattungen Methanobacterium, *Methanosarcina* und *Methanospiril*lum in den folgenden Reaktion zu Methan, Kohlendioxid und Wasser abgebaut:

1) CH₃COO- + H⁺ - - - - - - > CH₄ + CO₂

2) HCO₃- + H⁺ + 4H₂ - - > CH₄ + 3 H₂O

3) HCOO⁻ + H⁺ + 3H₂ - - > CH₄ + 2 H₂O

4) CH₃OH + H₂ - - - - - - > CH₄ + H₂O

Über 70% des Methans entstehen über die Spaltung von Essigsäure, also über Reaktion 1.

Da es sich bei der Methangasvergärung um einen Mischprozess handelt, bei dem in den verschiedenen Phasen verschiedene Mikroorganismen aktiv sind, muss den unterschiedlichen Ansprüchen aller Mikroorganismen Rechnung getragen werden, um einen möglichst hohen Ertrag zu erzielen. Ausschlaggebend sind jedoch die für die Aktivität der methanogenen Bakterien erforderlichen Bedingungen.

Letztere benötigen aufgrund ihrer Eigenschaften als obligate Anaerobier ein strikt sauerstoff-freies Milieu. Überdies bevorzugen sie einen leicht alkalischen pH-Wert.

In diesem Zusammenhang ist die Tatsache von Bedeutung, dass biologische Systeme unter Anoxie grundsätzlich zur Versauerung neigen. Dies ist dem Umstand geschuldet, dass viele Mikroorganismen bei Abwesenheit von Sauerstoff auf glykolytische Energiegewinnung umschalten, wobei große Mengen saurer Metabolite wie z.B. Laktat entstehen, die zu einer Versauerung des Milieus beitragen. Auch bei der Methangasvergärung entstehen in der ersten und zweiten Phase saure Metaboliten (Essigsäure, Ameisensäure), die zur einer Herabsenkung des pH-Wertes führen.

Hingegen stellt Gülle ein anoxisches System mit relativ hohem pH-Wert dar. Gülle ist aus diesem Grunde sehr gut geeignet, in einem Biogasfermenter die für Methanbakterien erforderlichen Bedingungen zu schaffen.

Der Feststoffgehalt sollte, da Feststoffe als Ansiedlungs- und Kontaktfläche für die methanogenen Bakterien dienen, nicht zu niedrig, aber auch nicht zu hoch sein. Als geeignet hat sich ein Feststoffgehalt im Bereich zwischen 2 - 9 % herausgestellt.

Die Temperatur sollte im Bereich zwischen 30 °C und 60 °C liegen. In vielen Fällen muss ein Biogasfermenter daher temperiert werden. Viele Heizeinrichtungen, z.B. im Fermenter angeordnete Wärmetauscher, weisen an ihrer Oberfläche eine höhere Temperatur auf. Gärmaterial, das mit der Heizeinrichtung in Berührung kommt, wird daher zunächst auf eine oberhalb des bevorzugten Temperaturbereichs liegende Temperatur erwärmt, und gibt diese dann sukzessive an das umgebende Material weiter. Auf diese Weise lässt sich zwar im gesamten Gärraum die gewünschte Temperatur einstellen, allerdings führt die erhöhte Temperatur im Bereich der Heizeinrichtung zum Absterben der dort angesiedelten methanogenen Bakterien, und damit zu einer Verminderung des Ertrags.

Zur gleichmäßigen Nährstoffversorgung, optimalen Raumausnutzung und zur Verhinderung der Anreicherung von toxischen Zwischenprodukten muß für eine gute Durchmischung gesorgt werden. Zusätzlich kann so der Bildung einer Schwimmschicht aus Fetten und freien Fettsäuren vorgebeugt werden, die einerseits die Entgasung verhindert und andererseits nicht als Substrat für die Methanogenese zur Verfügung steht

Aus der DE 197 564 85 ist ein Faulbehälter mit Rührwerk zum Einsatz in landwirtschaftlichen Biogasanlagen und kommunalen Kläranlagen bekannt. Dieser weist eine runde Bodenfläche, einen Füllstutzen und ein am Umfang des Faulbehälters angebrachtes Rührwerk mit einer Antriebsachse auf. Das Rührwerk ist in einem unterhalb des Füllstutzens angeordneten Rührrohr untergebracht. Das Rührrohr verläuft bevorzugt senkrecht. Der Inhalt des Gärbehälters wird über eine Wandheizung temperiert. Zu vergärendes Substrat wird über einen relativ weit oben angeordneten Füllstutzen in den Gärbehälter eingebracht, während über einen sehr viel weiter unten angeordneten Ablauf ausgegorenes, weiter unten im Behälter befindliches Material abgepumpt und in ein Gärrückstandslager verbracht wird.

Aufgrund verschiedener Umstände bietet der beschriebene Faulbehälter noch keine optimalen Lebensbedingungen für methanogene Bakterien. So wird zum Beispiel die Ausbildung einer Schwämmschicht nicht zuverlässig verhindert. Weiterhin führt die Anordnung einer Heizung im Bereich der Wand zu unerwünschten Temperaturgradienten, der wie oben erwähnt im Wandbereich zu einer Reduktion der Aktivität der Bakterien führt. Dasselbe gilt im Übrigen für Wärmetauscher oder Heizungen, die im Inneren eines Gärraums angeordnet sind. Außerdem ist das durch den Ablauf entnommene und ins GärRückstandslager verbrachte Material noch nicht vollständig ausgegoren. Da Gärrückstandslager in der Regel keine kontrollierten Bedingungen aufweisen und außerdem auch keine Gasauffangvorrichtung aufweisen, geht hier ein Teil des möglichen Ertrags verloren.

Aufgabe der vorliegenden Erfindung ist es, einen Fermenter zur Erzeugung von Biogas aus organischem Material bereitzustellen, der im Vergleich zu Vorrichtungen aus dem Stand der Technik höhere Erträge aufweist. Diese Aufgabe wird mit den Merkmalen des vorliegenden unabhängigen Vorrichtungsanspruchs gelöst.

Weitere Aufgabe der vorliegenden Erfindung ist es, ein Verfahren zur Erzeugung von Biogas aus organischem Material zur Verfügung zu stellen, das höhere Erträge als aus dem Stand der Technik bekannte Verfahren verspricht.

Diese Aufgabe wird mit den Merkmalen des vorliegenden unabhängigen Verfahrensanspruchs gelöst.

Gemäß unabhängigem Vorrichtungsanspruch ist ein Fermenter zur Erzeugung von Biogas aus organischem Material vorgesehen, aufweisend einen Gärraum mit im wesentlichen runder Grundfläche zur Aufnahme von Gärmaterial, eine im Randbereich des Gärraums angeordnete Einfülleinrichtung für zu vergärendes Substrat, einen über dem Gärraum angeordneten drucklosen Gasspeicher mit Gasentnahmeeinrichtung sowie eine Rühreinrichtung.

Ferner weist der Fermenter einen Beruhigungsraum mit Überlaufkante sowie eine Pumpeinrichtung zur kontinuierlichen oder schubweisen Entnahme von Gärmaterial aus dem Gärraum und Einbringung in den Beruhigungsraum auf.

Der Beruhigungsraum ist so ausgestaltet, dass sich das aus dem Gärraum eingebrachte Gärmaterial beruhigen und die aktive Biomasse, d.h. lebende Mikroorganismen, insbesondere Methanbakterien, sowie noch zu verstoffwechselnde Substrate, insbesondere methanogene Substrate, sich nach oben absetzen kann, während sich das weitgehend ausgegorene Material (passive Biomasse) nach unten absetzt.

Wie weiter unten beschrieben, bietet sich so die Möglichkeit, die aktive Biomasse, die in Vorrichtungen nach dem Stand der Technik zusammen mit dem ausgegorenen Material in ein Gärrückstandslager verbracht wird und dort ungenutzt verbleibt, zurückzugewinnen und dem Gärprozess erneut zuzuführen. Auf diese Weise wird der Ertrag wesentlich erhöht.

Hinzu kommt, dass durch die Rückführung der aktiven Biomasse den Gärraum der Zeitraum zur Optimierung der Anlage im Erstbetrieb wesentlich verkürzt wird. Grundsätzlich ist es so, dass eine Biogasanlage eine gewisse Zeit zur Optimierung braucht. Ursache hierfür ist, dass sich in der Anlage zunächst eine stabile Mikroorganismen-Flora einstellen muss. Durch die Möglichkeit, die in dem aus dem Gärraum entnommenen, durchgegorenenen Material noch befindlichen Mikroorganismen zurück zu gewinnen, wird der Zeitraum bis zum Aufbau einer stabilen und Mikroflora erheblich verkürzt. Auf diese Weise wird der maximale Ertrag also wesentlich schneller erreicht.

Die Möglichkeit der Rückführung der aktiven Biomasse weist noch einen anderen Vorteil auf: Da die Besatzdichte an aktiven Mikroorganismen im Gärraum auf einem wesentlich höheren Niveau gehalten werden kann, wird der Gärprozess beschleunigt. Daher kann der Durchsatz des Fermenters erhöht werden. So kann ein erfindungsgemäßer Fermenter wesentlich höhere Raumbelastungen tolerieren.

Es wird im Allgemeinen davon ausgegangen, dass im Gärraum eine oTS-Raumbelastung (organische Trockensubstanz) von ca. 1,5 bis 2,0 kg m⁻³ d⁻¹ nicht deutlich überschritten werden sollte, da höhere Raumbelastungen den Ertrag vermindern. Mithilfe der genannten Maßnahmen sind in dem erfindungsgemäßen Fermenter jedoch weitaus höhere Raumbelastungen möglich. So weist der erfindungsgemäße Fermenter nicht nur einen besseren Ertrag pro Tonne eingesetzten Materials auf, sondern ermöglicht auch einen höheren Durchsatz pro Zeit. beide Faktoren tragen wesentlich zu einer erheblich verbesserten Wirtschaftlichkeit des erfindungsgemäßen Fermenters bei.

Bevorzugt ist dabei einerseits vorgesehen, dass das Gärmaterial im unteren Bereich des Gärraums entnommen wird. Auf diese Weise wird sichergestellt, dass der Gehalt an aktiver Biomasse in dem dem Gärraum entnommenen Gärmaterial bereits reduziert ist.

Andererseits ist bevorzugt vorgesehen, dass das Gärmaterial nicht von oben in den Beruhigungsraum eingebracht wird, sondern in einem unterhalb von der Überlaufkante gelegenen Bereich. Auf diese Weise wird beim Einbringen des Gärmaterials in den Beruhigungsraum die sich in dessen oberem Bereich abgesetzte aktive Biomasse nach oben verdrängt wird und über die Überlaufkante abläuft, so dass sie dem Gärprozess im Gärraum erneut zugeführt werden kann.

In einer weiteren bevorzugten Ausgestaltung der erfindungsgemäßen Vorrichtung ist vorgesehen, dass der Gärraum in Form eines Ringkanals ausgeführt ist.

Ein solchermaßen ausgeführter Gärraum ist technisch gut beherrschbar. Mit entsprechenden Rührwerken lässt sich ein gut zu kontrollierender circumferentialer Materialstrom etablieren, und das Auftreten von Sink- und Schwimmschichten ist gut kontrollierbar.

In einer besonders bevorzugten Ausgestaltung ist vorgesehen, dass der Beruhigungsraum in seinem unteren Bereich einen Festbettreaktor aus Festmaterial aufweist.

Bei diesem Festmaterial kann es sich z.B. um poröses Material handeln, wie z.B. eine Schüttung aus Lavagranulat oder Blähtonkörnern. Ebenso geeignet ist eine Kunststoffmatrix, z.B. bestehend aus Kunststoffgranulat. Wichtig bei diesem Festbettreaktor ist, dass er einerseits nach unten hin durchlässig ist, und andererseits eine große innere Oberfläche aufweist, damit sich möglichst viele Mikroorganismen ansiedeln.

Der Festbettreaktor nimmt sukzessive das von oben eingetragene ausgegorene Material und bringt es in engen Kontakt mit seiner dicht von Mikroorganismen besiedelten inneren Oberfläche. Gleichzeitig wird der Materialdurchsatz stark verlangsamt. Auf diese Weise können auch im Material verbliebene Restbestände an organischen Kohlenstoffverbindungen vergoren werden, und der Ertrag des erfindungsgemäßen Fermenters wird weiter verbessert.

Bevorzugt ist unterhalb des Festbettreaktors eine Pumpeinrichtung zur Entnahme von ausgegorenem Restmaterial vorgesehen. Dieses kann in ein Gärrückstandslager verbracht werden, ist vollkommen ausgegoren und enthält daher praktisch keine organischen Bestandteile mehr. Er ist zudem wesentlich homogener strukturiert als herkömmliche Gärrückstände und weist gleichmäßigere und gleichbleibende Eigenschaften auf. Zudem ist er weniger stark mit Sink- und Schwimmstoffen belastet. Es kann daher insbesondere als Dünger verwendet werden.

In einer besonders bevorzugten Ausgestaltung ist vorgesehen, dass das Volumen des in den Beruhigungsraum eingebrachten Gärmaterials größer ist als das Volumen des aus dem Festbettreaktor entnommenen Restmaterials.

Auf diese Weise wird beim Einbringen des Gärmaterials in den Beruhigungsraum die dort oben abgesetzte aktive Biomasse nach oben verdrängt und läuft über die Überlaufkante ab, so dass sie dem Gärprozess im Gärraum erneut zugeführt werden kann.

Durch eine kegelförmige Ausgestaltung der Oberfläche des Festbettreaktors kann der Auswurf der aktiven Biomasse in den Gärraum weiter verbessert werden.

Besonders bevorzugt ist weiterhin vorgesehen, dass der Beruhigungsraum in der Mitte des Gärraums angeordnet ist, gleichermaßen im Zentrum des als Ringkanal ausgeführten Gärraums. Diese Ausgestaltung hat viele thermische, vorrichtungstechnische und verfahrenstechnische Vorteile. Der Beruhigungsraum kann aber, wenn gewünscht oder erforderlich, auch außerhalb des Gärraums angeordnet sein

Die Rühreinrichtung ist bevorzugt im Randbereich des Gärraums angeordnet. Diese Anordnung ermöglicht die effektive Erzeugung eines circumferentialen Materialstroms und eine hohe Effizienz der Durchmischung. Vorgesehen kann sein, dass die Förderrichtung in mehr oder weniger regelmäßigen Abständen umgekehrt wird, z.B. einmal täglich, um Verstopfungen im System vorzubeugen und Ablagerungen zu resuspendieren.

Grundsätzlich neigen die oben erwähnten Zwischenprodukte und methanogenen Substrate aufgrund ihres geringeren spezifischen Gewichts dazu, in der Gärmasse nach oben zu steigen und eine Schwimmschicht auszubilden. Diese Stoffe werden so der Metabolisierung durch die genannten Mikroorganismen, insbesondere Methanbakterien entzogen, was den Ertrag des Fermenters reduziert.

In einer besonders bevorzugten Ausgestaltung der erfindungsgemäßen Vorrichtung ist die Rühreinrichtung daher so ausgestaltet, dass sie Material von der Oberfläche der Gärmasse ansaugt und in Schrägrichtung nach unten presst, und zwar dergestalt, dass ein im Bereich der Rühreinrichtung sigmoid verlaufender Materialstrom erzeugbar ist. Auf diese Weise wird der Entstehung einer Schwimmschicht vorgebeugt, die Zwischenprodukte und methanogenen Substrate zurück in die Gärmasse befördert und den Mikroorganismen zugeführt. Der Ertrag des Fermenters wird durch diese Maßnahme wesentlich erhöht. Ebenso trägt auch diese Maßnahme zu einem schnelleren Erreichen der Optimalbedingungen im Fermenter bei und ermöglicht eine höhere oTS-Raumbelastung - mit den oben genannten Vorteilen.

Zudem können auf diese Weise auch solche Substrate effektiv vergoren werden, die stark zur Ausbildung von Schwimmschichten neigen und in herkömmlichen Fermentern ungern verwendet werden. Interessanterweise handelt es sich gerade bei diesen Substraten um sehr energiereiche Substrate, die potentiell einen sehr hohen Methanertrag versprechen. so z.B. um fettreiche Substrate wie Rapskuchen, Altfett, Flotatfett oder Backabfälle. Diese Substrate weisen Biogas-Bildungspotentziale zwischen 400 m³ t⁻¹ und 650 m³ t⁻¹ auf und liegen damit an der Spitze der in Frage kommenden Substrate.

Einige dieser Substrate sind, obwohl es sich um Abfallstoffe handelt, relativ teuer. So wird z.B. Rapskuchen auch gerne für die Tierernährung verwendet. Durch die beschriebene Annordnung können diese Substrate erstmals effektiv vergoren und damit ihr gesamtes Biogas-Bildungspotenzial ausgeschöpft werden, so dass sie - in Anbetracht des hohen Preises - erstmals wirtschaftlich in einer Biogasanlage als Substrat verwendet werden können.

Besonders bevorzugt besteht die Rühreinrichtung aus zwei Rührwerken. Diese weisen z.B. jeweils eine Antriebseinrichtung, eine Welle und einen Flügelpropeller auf, sind bevorzugt in ihrer Drehzahl variabel und können bezüglich ihres Winkels, d.h. z.B. des Winkels der Welle, zur Horizontalachse und/oder zur Vertikalachse verstellbar sein.

Dabei kann vorgesehen sein, dass eines der beiden Rührwerke schräg nach oben und das andere schräg nach unten weist, wobei beide Rührwerke jeweils an verschiedenen Seiten an der vertikalen Mittelachse des Gärraums vorbei weisen.

Mit Hilfe dieser Anordnung lässt sich der genannte, im Bereich der Rühreinrichtung sigmoid und ansonsten circumferential verlaufende Materialstrom gut einstellen.

Um den Materialstrom besser zu kontrollieren, können überdies im Randbereich des Gärraums Leitbleche vorgesehen sein.

Ebenso kann die Rühreinrichtung auch aus einem sich schräg an der Innenseite der Außenwand des Gärraums entlangziehenden Kanal mit nur einem Rührwerk bestehen. Auch dieser ist geeignet, einen im Bereich der Rühreinrichtung sigmoid und ansonsten circumferential verlaufende Materialstrom einstellen.

In einer besonders bevorzugten Ausgestaltung des erfindungsgemäßen Fermenters ist vorgesehen, dass der Fermenter eine Temperierungseinrichtung für das zu vergärende Substrat aufweist, die so eingerichtet ist, dass die Temperatur das Gärmaterials im Gärraum allein durch die Temperierung des über die Einfülleinrichtung eingebrachten, zu vergärenden Substrats einstellbar ist.

Hierfür ist neben einer Heizeinrichtung für das zu vergärende Substrat mindestens ein Temperaturfühler im Gärraum und ein entsprechender Regelkreis erforderlich.

Diese Art der Temperierung ist besonders effektiv, da das in den Gärraum eingebrachte, temperierte Material sich sofort verteilt und seine Temperatur schnell an die Umgebung abgibt. Da das zu vergärende Substrat noch keine Methanbakterien enthält, kann es durchaus auch auf eine Temperatur erwärmt werden, die über deren Temperaturoptimum liegt. Aufgrund der schnellen Temperaturabgabe an das umgebende Material werden die Methanbakterien im Fermenter nicht beeinträchtigt. Außerdem reicht aufgrund der guten thermischen Leitfähigkeit und der effektiven Durchmischung bereits eine geringfügig höhere Temperatur des zu vergärenden Substrats aus, den Fermenter effektiv zu temperieren, so dass auch aus diesem Grunde keine Beeinträchtigung der Methanbakterien im Fermenter zu befürchten ist. Insgesamt wird zudem eine schnellere und gleichmäßigere Temperierung des Gärmaterials ermöglicht, was der Prozessstabilität zugute kommt.

Dabei kann bevorzugt vorgesehen sein, dass die Einfülleinrichtung zwischen den beiden Rührwerken angeordnet ist. Das temperierte, zu ergärende Substrat wird auf diese Weise besonders effektiv in den Gärraum eingebracht und vermischt sich schnell mit dem Gärmaterial, wobei es seine Temperatur besonders schnell an die Umgebung abgibt.

Außerdem eröffnet sich so die Möglichkeit, das zu vergärende Substrat vor Einbringung in den Gärraum zu pasteurisieren bzw. zu sterilisieren. Auf diese Weise kann es nach Einbringung in den Gärraum besonders schnell von Methanbakterien besiedelt werden, was zu einer Forcierung der Gärung und damit zu einer Steigerung des Ertrags führt.

Diese Art der Temperierung erübrigt darüber hinaus das Vorhandensein weiterer Heizeinrichtungen oder Wärmetauscher im Gärraum und verhindert somit die oben genannten negativen Auswirkungen. Außerdem erübrigt diese Art der Temperierung das Vorhandensein von elektrischen Schaltungen im Gärraum, die ansonsten zu Funken- und damit Explosionsgefahr führen könnten.

In weiteren bevorzugten Ausgestaltungen ist vorgesehen, dass im Beruhigungsraum eine Einrichtung zur Verhinderung der Ausbildung einer Schwimmdecke an der Überlaufkante vorgesehen ist. Hierbei kann es sich z.B. um eine Drucklufteinrichtung handeln, über die von unten Luft oder Verdichtetes Biogas in den Beruhigungsraum eingeblasen wird. Die aufsteigenden Luftblasen zerreißen die Schwimmdecke und sorgen überdies dafür, dass aktive Biomasse über die Überlaufkante tritt und in den Gärraum zurückgeführt wird. Ebenso kann es sich bei der genannten Einrichtung um eine Abstreifeinrichtung handeln.

Der Gasspeicher des erfindungsgemäßen Fermenters besteht bevorzugt aus einer über den nach oben offenen Gärraum gespannten Folie. Diese hängt, solange sich noch nicht viel Gas entwickelt hat, relativ schlaff über dem Gärraum, wird dann aber durch das entstehende Gas nach oben verdrängt und gespannt. Das gebildete Gas kann dann auf bekannte Weise und mit bekannten Entnahmevorrichtungen entnommen werden. Besonders bevorzugt weist der Fermenter eine über dem Gasspeicher angeordnete Dachkonstruktion auf.

Bevorzugt ist weiterhin vorgesehen, dass im Bereich des Gärraums, des Gasspeichers und/oder des Beruhigungsraums keine elektrischen Einrichtungen vorgesehen sind. Ebenso kann der Gärraum, der Gasspeicher und/oder der Beruhigungsraum als Faradayscher Käfig ausgeführt sein. Beide Maßnahmen dienen der Feuer- und Explosionsverhütung.

Dazu kann das Gehäuse des Fermenters als Ganzes aus einem leitenden Metall sein (insbesondere V4A-Stahl), oder aber aus einem nichtmetallischen Material, dem ein Netz aus metallischen Leitern beigegeben ist, z.B. in Form eines das Gehäusematerial umgebenden Maschendrahtmaterials.

In einer weiteren bevorzugten Ausgestaltung weist der erfindungsgemäße Fermenter eine im Bodenbereich des Gärraums angeordnete Absetzrinne auf. In dieser kann sich anorganisches Material wie Sand, Kalk, Steine etc. absetzen und, beispielsweise mit Hilfe einer Förderschnecke, aus dem Fermenter entfernt werden. Üblicherweise werden täglich etwa 1 - 3% des Gärmaterials auf diese Weise entfernt. Feststoffe können dann aus dem ausgetragenen Material abgeschieden und die flüssigen Bestandteile wieder in den Gärraum zurückgeführt werden.

Es ist weiterhin ein Verfahren zur Erzeugung von Biogas aus organischem Material in einem Fermenter vorgesehen. Dieses weist die folgenden Schritte auf:
a) Einbringen von zu vergärendem Substrat durch eine Einfülleinrichtung in einen Gärraum mit im Wesentlichen runder Grundfläche;
b) Erzeugung eines circumferential gerichteten Materialstroms im Gärraum mittels einer Rühreinrichtung;
c) Erzeugung und Aufrechterhaltung eines anaeroben Milieus, eines pH-Werts von mindestens 7 und einer Temperatur im mesophilen bis thermophilen Bereich,
d) Auffangen des entstehenden Gases in einem druck-losen Gasspeicher und kontinuierliche oder schubweise Entnahme des aufgefangenen Gases; sowie
e) kontinuierliche oder schubweise Entnahme von Gärmaterial aus dem Gärraum und Einbringung in einen Beruhigungsraum.

Besonders bevorzugt liegt der pH-Wert im Bereich von einschließlich pH 7 bis einschließlich pH 8. Der erwähnte mesophile Temperaturbereich umfasst Temperaturen von einschließlich 30 °C bis einschließlich 45 °C. Der erwähnte thermophile Temperaturbereich umfasst hingegen Temperaturen von einschließlich 42 °C bis einschließlich 60°C. Ein besonders bevorzugter Temperaturbereich liegt zwischen einschließlich 35 °C und einschließlich 42 °C.

Bevorzugt ist dabei vorgesehen, dass der Materialstrom zusätzlich zu der circumferential gerichteten Bewegungskomponente in einem Teilbereich eine von der Oberfläche der Gärmasse nach unten gerichtete Bewegungskomponente aufweist. Bevorzugt ist weiterhin vorgesehen, dass sich im Beruhigungsraum sich aktive Biomasse nach oben absetzt und in den Gärraum zurückgeführt wird, während sich passive Biomasse nach unten absetzt.

Die Begriffe "aktive Biomasse" und "passive Biomasse" wurden weiter oben bereits definiert.

Die passive Biomasse gelangt bevorzugt in einen Festbettreaktor aus porösem Festmaterial, gärt dort weiter, wobei ausgegorenes Restmaterial, das den Festbettreaktor passiert hat, bevorzugt kontinuierlich oder schubweise entnommen und gelagert wird.

Besonders bevorzugt erfolgen die Entnahme von Gärmaterial aus dem Gärraum und dessen Einbringung in den Beruhigungsraum und die Entnahme von ausgegorenem Restmaterial aus dem Festbettreaktor synchronisiert. Dabei ist das Volumen des in den Beruhigungsraum eingebrachten Gärmaterials bevorzugt größer als das Volumen des aus dem Festbettreaktor entnommenen Restmaterials.

Auf diese Weise wird beim Einbringen des Gärmaterials in den Beruhigungsraum sichergestellt, dass die dort oben abgesetzte aktive Biomasse nach oben verdrängt wird und über die Überlaufkante abläuft, so dass sie dem Gärprozess im Gärraum erneut zugeführt werden kann.

Besonders bevorzugt ist das Volumen des in den Beruhigungsraum eingebrachten Gärmaterials doppelt so groß wie das Volumen des aus dem Festbettreaktor entnommenen Restmaterials.

Weiterhin ist bevorzugt vorgesehen, dass durch Einblasen von Druckluft oder verdichtetem Biogas in den Beruhigungsraum die Ausbildung einer Schwimmschicht an der Überlaufkante verhindert wird. Hier kann alternativ vorgesehen sein, dass die Ausbildung einer Schwimmschicht an der Überlaufkante durch Abstreifen mit einer geeigneten Einrichtung verhindert wird.

Auf diese Weise wird außerdem verhindert, dass die Funktion des Festbettreaktors durch Sink- oder Schwimmstoffe beeinträchtigt oder in Frage gestellt wird.

Besonders bevorzugt enthält das zu vergärende Substrat Komponenten ausgewählt aus der Gruppe bestehend aus Tierexkrementen und -fäkalien, biologischen Abfallstoffen, nachwachsenden Rohstoffen, und zur Fermentierung geeigneten Pflanzen und Pflanzenteilen.

Bei den Tierexkrementen und Fäkalien kann es sich z.B. um Gülle, Jauche oder Mist handeln. Bei den Biologischen Abfallstoffen handelt es sich z.B. um Reste aus der Lebensmittelproduktion, Kartoffelschlempe oder -Schalen, Presskuchen von Ölsaaten, Schlacht- und/oder Speiseabfälle, verdorbenes Obst, Gemüse und Lebensmittel, Tierstreu, Speisefette und Öle und dergleichen. Bei den nachwachsenden Rohstoffen handelt es sich z.B. um Maissilage, Grassilage, Weizen, Zuckerrübenschnitzel, Rapssaat und ähnliches. Bei zur Fermentierung geeigneten Pflanzen und Pflanzenteilen handelt es sich z.B. um Grasschnitt, Häckselabfälle, Stroh oder Mais- oder Rübengrün.

Gülle stellt ein anoxisches System mit relativ hohem pH-Wert dar und aus diesem Grunde sehr gut geeignet, in einem Biogasfermenter die für Methanbakterien erforderlichen Bedingungen zu schaffen.

Die Erfindung wird nachfolgend mit Bezug auf die Zeichnungen beispielhaft erläutert. Es werden dabei Ausführungsbeispiele gezeigt, die den Schutzbereich der vorgelegten Ansprüche in keiner Weise einschränken sollen. Es zeigen:
- Figur 1:: einen erfindungsgemäßen Fermenter zur Erzeu- gung von Biogas aus organischem Material im Querschnitt;
- Figur 2 und 3:: das Funktionsprinzip des erfindungsgemäßen Fermenters zur Erzeugung von Biogas; sowie
- Figur 4:: einen erfindungsgemäßen Fermenter in per- spektivischer Ansicht, aus dem der Strö- mungsverlauf des Gärmaterials hervorgeht.

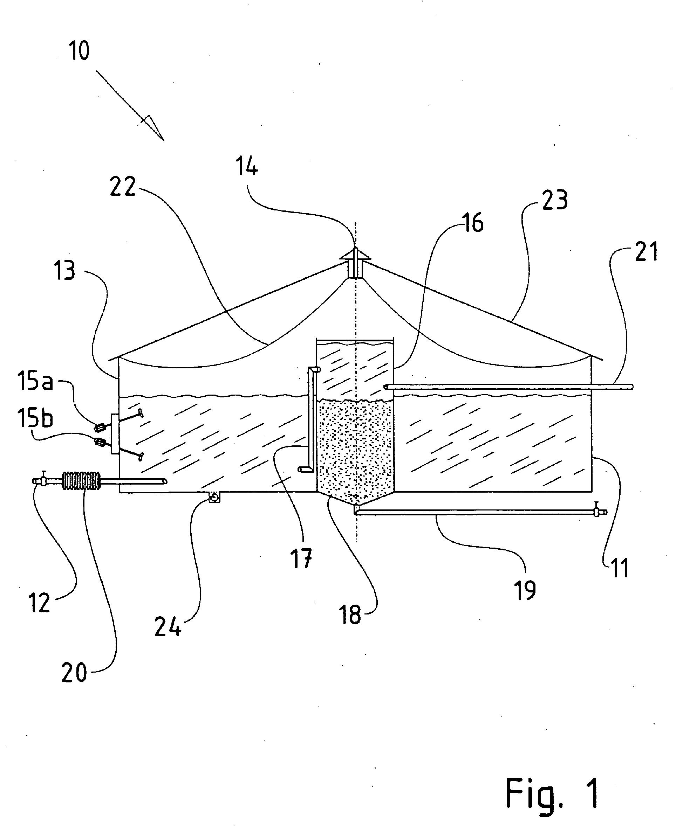

Figur 1 zeigt einen erfindungsgemäßen Fermenter 10 zur Erzeugung von Biogas aus organischem Material. Dieser weist einen Gärraum 11 mit im Wesentlichen runder Grundfläche auf, der zur Aufnahme von Gärmaterial (schräg schraffiert dargestellt) eingerichtet ist. Der Fermenter weist überdies eine im Randbereich des Gärraums angeordnete Einfülleinrichtung 12 für zu vergärendes Substrat auf, sowie einen über dem Gärraum angeordneten drucklosen Gasspeicher 13 mit Gasentnahmeeinrichtung 14. Im Randbereich des Gärraums angeordnet findet sich eine Rühreinrichtung 15, bestehend aus zwei Rührwerken 15 a, 15 b, von denen eines schräg nach oben und das andere schräg nach unten weist. Nicht erkennbar in Figur 1 ist, dass beide Rührwerke jeweils an verschiedenen Seiten an der gestrichelt dargestellten Mittelachse des Gärraums 10 vorbei weisen.

Weiterhin weist der Fermenter einen Beruhigungsraum 16 mit einer Überlaufkante auf, sowie eine Pumpeinrichtung 17 zur kontinuierlichen oder schubweisen Entnahme von Gärmaterial aus dem Gärraum und Einbringung in den Beruhigungsraum. Der Beruhigungsraum 16 ist in der Mitte des einen im Wesentlichen runde Grundfläche aufweisenden Gärraums 11. angeordnet, so dass der Gärraum in Form eines Ringkanals ausgeführt ist.

Der Beruhigungsraum weist in seinem unteren Bereich einen Festbettreaktor 18 aus porösem Festmaterial auf, sowie eine Pumpeinrichtung 19 zur Entnahme von ausgegorenem Restmaterial aus dem Festbettreaktor. Weiterhin weist der Fermenter eine Temperierungseinrichtung 20 für das zu vergärende Substrat auf, die im Bereich der Einfülleinrichtung 12 angeordnet ist. Ferner verfügt der Fermenter über eine Drucklufteinrichtung 21 zum Einblasen von Druckluft in den Beruhigungsraum 16, sowie eine Dachkonstruktion 23, an der eine den Gasspeicher nach oben hin begrenzende Folie 22 aufgehängt ist. Ferner weist der Fermenter eine im Bodenbereich des Gärraums angeordnete Absetzrinne 24 auf.

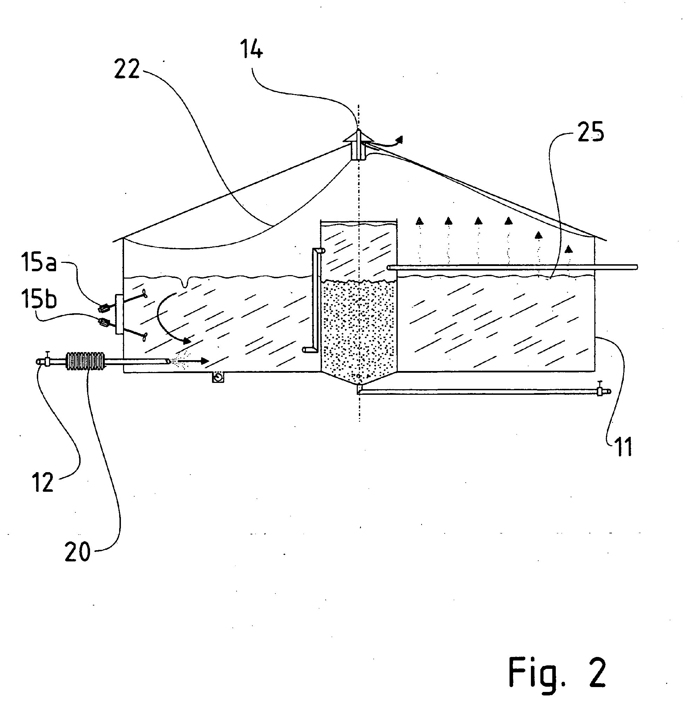

Figur 2 zeigt verschiedene Funktionsprinzipien des erfinungsgemäßen Fermenters. Durch die Einfülleinrichtung 12 wird mittels der Temperiereinrichtung 20 vortemperiertes zu vergärendes Substrat in den Gärraum 11 eingebracht. Das im Gärraum befindliche Gärmaterial wird mithilfe der Rühreinrichtung 15 umgerührt, wobei sich ein circumferentialer Materialstrom einstellt. In der rechts der vertikalen Achse gezeigten Hälfte des Fermenters ist erkennbar, dass bereits der Gärprozess eingesetzt hat und gebildetes Biogas nach oben hin entweicht. Die in der linken Hälfte des Fermenters noch durchhängend dargestellte Folie 22 wird in der rechten Hälfte des Fermenters bereits nach oben hin verdrängt und verdrängt ihrerseits oberhalb angeordnete Luft über eine im Bereich der Gasentnahmeeinrichtung angeordnete Durchführung. Weiterhin ist in Figur 2 eine Schwimmschicht 25 gezeigt, die aus Zwischenprodukten und methanogenen Substraten besteht, die aufgrund ihres geringeren spezifischen Gewichtes in der Gärmasse nach oben steigen. Diese Stoffe werden der Metabolisierung durch die genannten Mikroorganismen, insbesondere durch die Methanbakterien entzogen, was den Ertrag des Fermenters reduziert. In der linken Hälfte des in Figur 2 dargestellten Fermenters ist gezeigt, dass durch die Aktivität der Rühreinrichtung 15 ein nach unten gerichteter Materialstrom erzeugt wird, der die Schwimmschicht aufreißt und das in der Schwimmschicht befindliche Material nach unten befördert, wo es von den genannten Mikroorganismen verstoffwechselt wird.

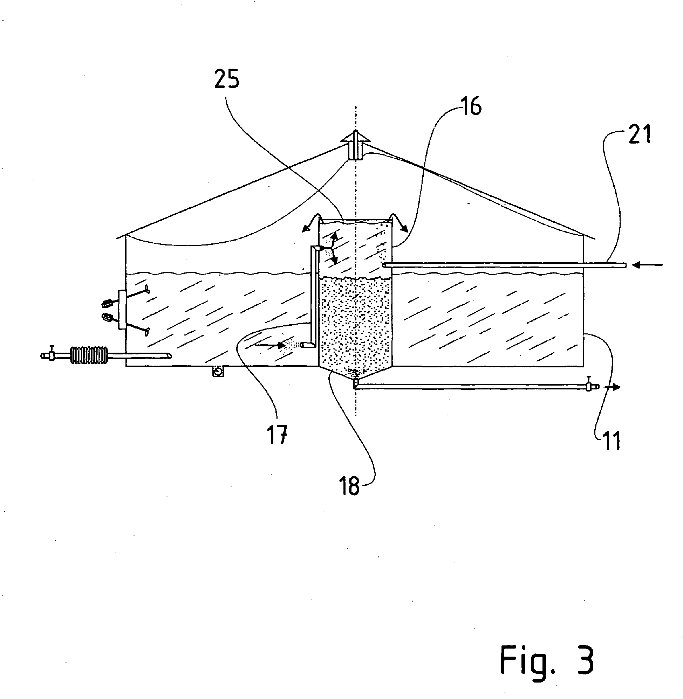

In Figur 3 ist gezeigt, dass Gärmaterial aus dem Gärraum 11 über die Pumpeinrichtung 17 in den Beruhigungsraum 16 befördert wird. Das Gärmaterial wird im unteren Bereich des Gärraums entnommen, so dass der Gehalt an aktiver Biomasse bereits reduziert ist, und in einem unterhalb von der Überlaufkante des Beruhigungsraums 16 gelegenen Bereichs in den Beruhigungsraums 16 eingebracht, so dass beim Enbringen des Gärmaterials in den Beruhigungsraum die dort oben abgesetzte Schwimmschicht 25 nach oben verdrängt wird und über die Überlaufkante abläuft. Auf diese Weise kann die aktive Biomasse, die insbesondere Mikroorganismen sowie methanogene Substrate enthält, zurückgewonnen werden. Diese Maßnahme erhöht den Ertrag, trägt zu einem schnelleren Erreichen der Optimalbedingungen im Fermenter bei und ermöglicht überdies eine höhere oTS-Raumbelastung - mit den oben genannten Vorteilen.

Weitgehend ausgegorenes Material (passive Biomasse) setzt sich hingegen im Beruhigungsraum 16 nach unten ab und gelangt in den Festbettreaktor 18, der ein poröses Material enthält, zum Beispiel eine Schüttung aus Lava-Granulat oder Blähtonkörnern. Dieses Material weist eine große innere Oberfläche auf und bietet damit viele Ansiedlungsmöglichkeiten für Mikroorganismen, die für eine vollständige Ausgärung des Materials sorgen.

Über die Pumpeinrichtung 19 wird ausgegorenes Material aus dem Festbettreaktor entnommen. Das Volumen des in den Beruhigungsraum eingebrachten Gärmaterials ist dabei bevorzugt größer als das Volumen des aus dem Festbettreaktor entnommenen Materials. Auf diese Weise wird sichergestellt, dass beim Einbringen des Gärmaterials in den Beruhigungsraum die dort oben abgesetzte aktive Biomasse nach oben verdrängt wird und über die Überlaufkante abläuft, so dass sie dem Gärprozess im Gärraum wieder zugeführt werden kann.

Über die Druckluftleitung 21 wird kontinuierlich oder schubweise Druckluft in den Beruhigungsraum eingeblasen.

Die aufsteigenden Luftblasen zerreißen die Schwimmdecke und sorgen ebenfalls dafür, dass aktive Biomasse über die Überlaufkante tritt und in den Gärraum zurückgeführt wird.

Durch eine in Fig. 3 nicht gezeigte kegelförmige Ausgestaltung der Oberfläche des Festbettreaktors kann der Auswurf der aktiven Biomasse in den Gärraum überdies weiter verbessert werden.

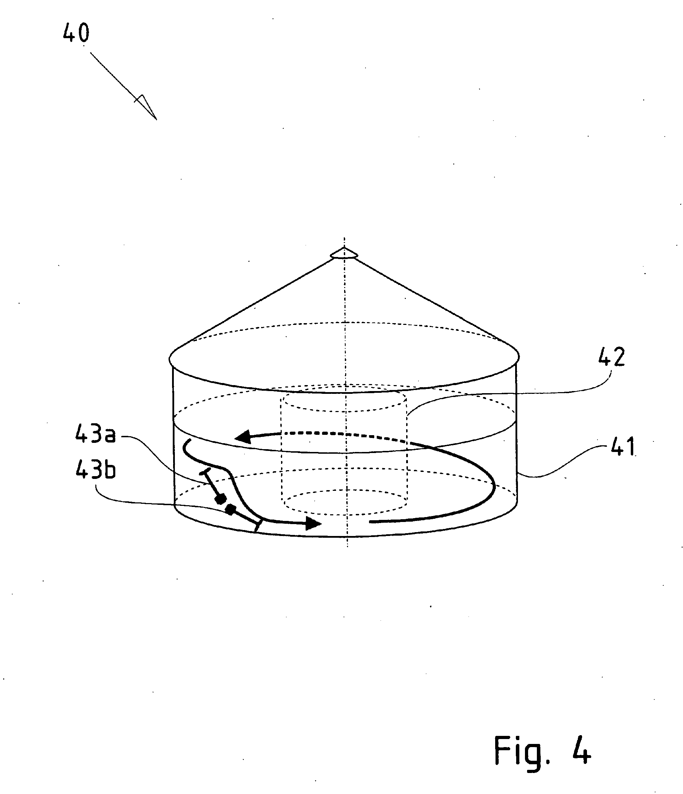

Figur 4 zeigt einen erfindungsgemäßen Fermenter 40 in perspektivischer Ansicht mit einem Gärraum 41, einem Beruhigungsraum 42 sowie einer Rühreinrichtung 43. Die Rühreinrichtung besteht aus zwei Rührwerken 43 a und 43 b, von denen das eine schräg nach oben und das andere schräg nach unten weist, wobei beide Rührwerke jeweils an verschiedenen Seiten an der vertikalen Mittelachse des Gärraum vorbei weisen. Auf diese Weise lässt sich ein Materialstrom etablieren, der im Bereich der Rühreinrichtung einen von oben nach unten gerichteten, sigmoidalen Verlauf aufweist, und ansonsten circumferential den in Form eines Ringkanals ausgeführten Gärraum 41 durchläuft. Auf diese Weise wird einerseits für eine gute Durchmischung des Gärmaterials gesorgt und andererseits der Entstehung einer Schwimmschicht vorgebeugt, so dass die Zwischenprodukte und methanogenen Substrate zurück in die Gärmasse befördert und den Mikroorganismen zugeführt werden. Diese Maßnahme erhöht den Ertrag, trägt zu einem schnelleren Erreichen der Optimalbedingungen im Fermenter bei und ermöglicht überdies eine höhere oTS-Raumbelastung - mit den oben genannten Vorteilen.

## Patentansprüche

1. Fermenter (10) zur Erzeugung von Biogas aus organischem Material, aufweisend:
a) einen Gärraum (11) mit im wesentlichen runder Grundfläche zur Aufnahme von Gärmaterial;
b) eine im Randbereich des Gärraums angeordnete Einfülleinrichtung (12) für zu vergärendes Substrat;
c) einen über dem Gärraum angeordneten drucklosen Gasspeicher (13) mit Gasentnahmeeinrichtung (14);
d) eine Rühreinrichtung (15);
e) einen Beruhigungsraum (16) mit Überlaufkante; sowie eine Pumpeinrichtung (17) zur kontinuierlichen oder schubweisen Entnahme von Gärmaterial aus dem Gärraum und Einbringung in den Beruhigungsraum.

2. Fermenter gemäss Anspruch 1, **dadurch gekennzeichnet, dass** der Gärraum in Form eines Ringkanals ausgeführt ist.

3. Fermenter gemäss einem der vorherigen Ansprüche, **dadurch gekennzeichnet, dass** der Beruhigungsraum in seinem unteren Bereich einen Festbettreaktor (18) aus Festmaterial aufweist.

4. Fermenter gemäss Anspruch 3, **dadurch gekennzeichnet, dass** unterhalb des Festbettreaktors eine Pumpeinrichtung (19) zur Entnahme von ausgegorenem Restmaterial vorgesehen ist.

5. Fermenter gemäss einem der vorherigen Ansprüche, **dadurch gekennzeichnet, dass** der Beruhigungsraum in der Mitte des Gärraum angeordnet ist.

6. Fermenter gemäss einem der vorherigen Ansprüche, **dadurch gekennzeichnet, dass** die Rühreinrichtung so ausgestaltet ist, dass sie Material von der Oberfläche der Gärmasse ansaugt und in Schrägrichtung nach unten presst, dergestalt, dass ein im Bereich der Rühreinrichtung sigmoid verlaufender Materialstrom erzeugbar ist, der ansonsten circumferential den Gärraum durchläuft.

7. Fermenter gemäss einem der vorherigen Ansprüche, **dadurch gekennzeichnet, dass** der Gasspeicher nach oben hin durch eine über den nach oben offenen Gärraum gespannte Folie (22) begrenzt ist.

8. Fermenter gemäss einem der vorherigen Ansprüche, **dadurch gekennzeichnet, dass** der Fermenter eine über dem Gasspeicher angeordnete Dachkonstruktion (23) aufweist.

9. Fermenter gemäss einem der vorherigen Ansprüche, **dadurch gekennzeichnet, dass** im Bereich des Gärraums, des Gasspeichers und/oder des Beruhigungsraums keine elektrischen Einrichtungen vorgesehen sind.

10. Fermenter gemäss einem der vorherigen Ansprüche, **dadurch gekennzeichnet, dass** der Fermenter eine im Bodenbereich des Gärraums angeordnete Absetzrinne (24) aufweist.

11. Verfahren zur Erzeugung von Biogas aus organischem Material in einem Fermenter, aufweisend die folgenden Schritte:
a) Einbringen von zu vergärendem Substrat durch eine Einfülleinrichtung in einen Gärraum mit im wesentlichen runder Grundfläche;
b) Erzeugung eines circumferential gerichteten Materialstroms im Gärraum mittels einer Rühreinrichtung;
c) Erzeugung und Aufrechterhaltung eines anaeroben Milieus, eines pH-Werts von mindestens 7 und einer Temperatur im mesophilen bis thermophilen Bereich;
d) Auffangen des entstehenden Gases in einem drucklosen Gasspeicher und kontinuierliche oder schubweise Entnahme des aufgefangenen Gases,- sowie
e) kontinuierliche oder schubweise Entnahme von Gärmaterial aus dem Gärraum und Einbringung in einen Beruhigungsraum.

12. Verfahren gemäss Anspruch 11, **dadurch gekennzeichnet, dass** der Materialstrom zusätzlich zu der circumferential gerichteten Bewegungskomponente in einem Teilbereich eine von der Oberfläche der Gärmasse nach unten gerichtete Bewegungskomponente aufweist.

13. Verfahren gemäss einem der Ansprüche 11 - 12, **dadurch gekennzeichnet, dass** sich in dem Beruhigungsraum aktive Biomasse nach oben absetzt und in den Gärraum zurückgeführt wird, während sich passive Biomasse nach unten absetzt.

14. Verfahren gemäss einem der Ansprüche 11- 13, **dadurch gekennzeichnet, dass** die passive Biomasse in einen Festbettreaktor aus porösem Festmaterial gelangt.

15. Verfahren gemäss einem der Ansprüche 11 - 14, **dadurch gekennzeichnet, dass** ausgegorenes Restmaterial, das den Festbettreaktor passiert hat, kontinuierlich oder schubweise entnommen und gelagert wird.

16. Verfahren gemäss einem der Ansprüche 11 - 15, **dadurch gekennzeichnet, dass** das Volumen des in den Beruhigungsraum eingebrachten Gärmaterials grösser ist als das Volumen des aus dem Festbettreaktor entnommenen Restmaterials.

17. Verfahren gemäss einem der Ansprüche 11 - 16, **dadurch gekennzeichnet, dass** das zu vergärende Substrat Komponenten enthält ausgewählt aus der Gruppe bestehend aus Tierexkrementen und -fäkalien, biologische Abfallstoffen, nachwachsenden Rohstoffen, und zur Fermentierung geeigneten Pflanzen und Pflanzenteilen.

## Claims

1. A fermenter (10) for producing biogas from organic material, having:
a) a fermentation chamber (11) with a essentially round base surface to receive fermentation material;
b) arranged, in the peripheral region of the fermentation chamber, a filling mean (12) for substrate to be fermented;
c) arranged, above the fermentation chamber, an unpressurized gas store (13) with a gas discharging mean (14);
d) a stirring mean (15);
e) a settling chamber (16) with overflow rim; and also a pumping mean (17) for the continuous or batchwise removal of fermentation material from the fermentation chamber and introduction into the settling chamber.

2. The fermenter according to claim 1, **characterized in that** the fermentation chamber has been designed in the form of an annular channel.

3. The fermenter according to any of the preceding claims, **characterized in that** the settling chamber has, in its lower region, a fixed-bed reactor (18) composed of solid material.

4. The fermenter according to claim 3, **characterized in that**, below the fixed-bed reactor, there is a pumping mean (19) provided for the removal of remaining exhausted fermented material.

5. The fermenter according to any of the preceding claims, **characterized in that** the settling chamber has been arranged in the center of the fermentation chamber.

6. The fermenter according to any of the preceding claims, **characterized in that** the stirring mean has been arranged in the peripheral region of the fermentation chamber.

7. The fermented as according to any of the preceding claims, **characterized in that** a film (22) stretched over the open upper side of the fermentation chamber limits the upper side of the gas store.

8. The fermenter according to any of the preceding claims, **characterized in that** the fermenter has a roof structure (23) arranged over the gas store.

9. The fermenter according to any of the preceding claims, **characterized in that** there is no electric equipment provided in the region of the fermentation chamber, of the gas store, and/or of the settling chamber.

10. The fermenter according to any of the preceding claims, **characterized in that** the fermenter has a sludge gutter (24) arranged in the floor region of the fermentation chamber,

11. A process for producing biogas from organic material in a fermented, having the following steps:
a) introduction of substrate to be fermented, via filling means, into a fermentation chamber with a essentially round base surface;
b) generation of a circumferentially directed stream of material in the fermentation chamber by means of a stirring mean;
c) generation and maintenance of an anaerobic environment, of a pH of at least 7, and of a temperature in the mesophilic to thermophilic range;
d) collection, in an unpressurized gas store, of the gas produced, and continuous or batchwise removal of the collected gas; and also
e) continuous or batchwise removal of fermentation material from the fermentation chamber and introduction into a settling chamber.

12. The process as claimed in claim 11, **characterized in that** the stream of material has, in addition to the circumferentially directed movement component, in a subregion, a movement component directed downward from the surface of the fermentation mass.

13. The process as claimed in either of claims 11 and 12, **characterized in that**, in the settling chamber, active biomass rises and is returned to the fermentation chamber, whereas passive biomass sinks.

14. The process as claimed in any of claims 11 to 13, **characterized in that** the passive biomass passes into a fixed-bed reactor composed of porous solid material.

15. The process as claimed in any of claims 11 to 14, **characterized in that** exhausted fermented residual material which has passed through the fixed-bed reactor is removed and stored, continuously or batchwise.

16. The process as claimed in any of claims 11 to 15, **characterized in that** the volume of the fermentation material introduced into the settling chamber is greater than the
volume of the residual material removed from the fixed-bed reactor.

17. The process as claimed in any of claims 11 to 16, **characterized in that** the substrate to be fermented comprises components selected from the group consisting of animal excrement and animal feces, biological waste, renewable raw materials, and fermentable plants and plant parts.

## Revendications

1. Fermenteur (10) pour la production de biogaz à partir de matière organique, comprenant :
a) une chambre de fermentation (11) à surface de base essentiellement ronde, destinée à recevoir la matière à fermenter ;
b) un dispositif d'introduction (12) du substrat de fermentation situé à la périphérie de la chambre de fermentation ;
c) un réservoir de gaz sans pression (13) disposé au-dessus de la chambre de fermentation et pourvu d'un dispositif de prélèvement de gaz (14) ;
d) un dispositif de brassage (15) ;
e) une chambre de stabilisation (16) pourvue d'un bord de surverse ; ainsi qu'un dispositif de pompe (17) qui prélève la matière à fermenter dans la chambre de fermentation de manière continue ou par portions et l'alimente dans la chambre de stabilisation.

2. Fermenteur selon la revendication 1, **caractérisé en ce que** la chambre de fermentation est exécutée sous la forme d'un canal annulaire.

3. Fermenteur selon l'une des revendications précédentes, **caractérisé en ce que** la chambre de stabilisation comporte, dans sa zone inférieure, un réacteur à lit solide (18) formé d'un matériau solide.

4. Fermenteur selon la revendication 3, **caractérisé en ce qu'**un dispositif de pompe (19) permettant le retrait de la matière résiduelle fermentée est prévu en dessous du réacteur à lit solide.

5. Fermenteur selon l'une des revendications précédentes, **caractérisé en ce que** la chambre de stabilisation est située au centre de la chambre de fermentation.

6. Fermenteur selon l'une des revendications précédentes, **caractérisé en ce que** le dispositif de brassage est réalisé de manière à aspirer la matière de la surface de la masse en fermentation et à la pousser vers le bas dans une direction oblique de façon à pouvoir produire un courant de matière s'écoulant de manière sigmoïde dans la zone du dispositif de brassage, lequel courant s'écoule sinon de façon circonférentielle à travers la chambre de fermentation.

7. Fermenteur selon l'une des revendications précédentes, **caractérisé en ce que** le réservoir de gaz est limité, vers le haut, par une feuille (22) tendue au-dessus de la chambre de fermentation ouverte vers le haut.

8. Fermenteur selon l'une des revendications précédentes, **caractérisé en ce que** le fermenteur est pourvu d'une construction en toit (23) située au-dessus du réservoir de gaz.

9. Fermenteur selon l'une des revendications précédentes, **caractérisé en ce qu'**aucun dispositif électrique n'est prévu dans la zone de la chambre de fermentation, du réservoir de gaz et/ou de la chambre de stabilisation.

10. Fermenteur selon l'une quelconque des revendications précédentes, **caractérisé en ce que** le fermenteur est pourvu d'une goulotte de décantation (24) située dans la zone du fond de la chambre de fermentation.

11. Procédé de production de biogaz à partir de matière organique dans un fermenteur, comprenant les étapes suivantes :
a) alimentation d'un substrat de fermentation, au moyen d'un dispositif d'introduction, dans une chambre de fermentation à surface de base essentiellement ronde ;
b) production d'un courant de matière dans la direction circonférentielle dans la chambre de fermentation au moyen d'un dispositif de brassage ;
c) production et maintien d'un milieu anaérobie, d'un pH d'au moins 7 et d'une température allant du domaine mésophile au domaine thermophile ;
d) captage du gaz produit dans un réservoir de gaz sans pression et prélèvement du gaz capté de manière continue ou par portions, ainsi que
e) prélèvement de la matière à fermenter dans la chambre de fermentation de manière continue ou par portions et alimentation de la matière dans la chambre de stabilisation.

12. Procédé selon la revendication 11, **caractérisé en ce que** le courant de matière comprend, en plus de la composante de mouvement dans la direction circonférentielle, une composante de mouvement dirigée de la surface de la masse en fermentation vers le bas dans une partie du courant.

13. Procédé selon l'une des revendications 11-12, **caractérisé en ce que** la biomasse active se dépose vers le haut dans la chambre de stabilisation et est ramenée dans la chambre de fermentation, tandis que la biomasse passive se dépose vers le bas.

14. Procédé selon l'une des revendications 11 - 13, **caractérisé en ce que** la biomasse passive arrive dans un réacteur à lit solide formé d'un matériau solide poreux.

15. Procédé selon l'une des revendications 11 14, **caractérisé en ce que** la matière résiduelle fermentée qui a traversé le réacteur à lit solide est retirée de manière continue ou par portions et stockée.

16. Procédé selon l'une des revendications 11 - 15, **caractérisé en ce que** le volume de la matière à fermenter alimentée dans la chambre de stabilisation est supérieur au volume de la matière résiduelle retirée du réacteur à lit solide.

17. Procédé selon l'une des revendications 11-16, **caractérisé en ce que** le substrat de fermentation contient des composants choisis dans le groupe comprenant des excréments et des matières fécales d'animaux, des déchets biologiques, des matières premières renouvelables et des végétaux et parties de végétaux aptes à la fermentation.
